# EUROPÄISCHE PATENTSCHRIFT

(11) **EP 2 413 143 B1**
(45) Veröffentlichungstag und Bekanntmachung des Hinweises auf die Patenterteilung: **01.05.2013**
(21) Anmeldenummer: 11008623.8
(22) Anmeldetag: 24.11.2010
(51) Int. Cl.: G01N 33/86, G01N 33/96

(54) **Heterogener Gerinnungstest**
Heterogeneous coagulation test
Test de coagulation hétérogène

(30) Priorität: 09.12.2009 EP 09015240
(43) Veröffentlichungstag der Anmeldung: 01.02.2012
(62) Teilanmeldung aus: 10192331.6
(73) Patentinhaber: Siemens Healthcare Diagnostics Products GmbH, 35041 Marburg (DE)
(72) Erfinder: Christ, Gerlinde, 35043 Marburg (DE); Kappel, Andreas, 61462 Koenigstein (DE)

(56) Entgegenhaltungen:
- EP-A1- 0 034 320
- WO-A1-99/00515
- WO-A2-01/44493
- US-A- 5 348 942
- US-A1- 2005 238 536
- US-B2- 7 153 473
- MAHDI F ET AL: "Protease Nexin-2/Amyloid.beta.-Protein Precursor Inhibits Factor Xa in the Prothrombinase Complex", THE JOURNAL OF BIOLOGICAL CHEMISTRY, Bd. 270, Nr. 40, 6. Oktober 1995 (1995-10-06), Seiten 23468-23474, XP002582743,

## Beschreibung

Die vorliegende Erfindung liegt auf dem Gebiet der Gerinnungsdiagnostik und betrifft Verfahren zur Bestimmung von Antikoagulanzien, die die Aktivität von Blutgerinnungsfaktoren inhibieren.

In der Gerinnungsdiagnostik werden sogenannte Globalteste zur Untersuchung der Funktionalität der Blutgerinnungskaskade und sogenannte Einzelteste zur Bestimmung der Aktivität einzelner Blutgerinnungsfaktoren unterschieden. Sowohl für die Globalteste als auch für die Einzelteste sind unterschiedliche Testformate bekannt. In Bezug auf das Testformat werden im wesentlichen Gerinnungsteste und chromogene Teste unterschieden.

Bei einem Gerinnungstest wird die zu untersuchende Patientenprobe, die üblicherweise aus Plasma besteht, mit einem Gerinnungsaktivator vermischt, der den Gerinnungsprozess startet. Der Gerinnungsprozess führt zur Bildung eines Fibringerinnsels, das mit Hilfe photometrischer Verfahren gemessen werden kann. Die Fibrinbildungsgeschwindigkeit ist ein Maß für die Funktionalität der Blutgerinnungskaskade. Zur Bestimmung der Aktivität eines einzelnen Gerinnungsfaktors in einem Gerinnungstest wird die zu untersuchende Patientenprobe zusätzlich mit einem Mangelplasma vermischt, welches alle Komponenten der Blutgerinnungskaskade supplementiert außer den zu testenden Blutgerinnungsfaktor.

Bei einem chromogenen Test wird die zu untersuchende Patientenprobe, die üblicherweise aus Plasma besteht, mit einem Gerinnungsaktivator und mit einem Substrat für einen Gerinnungsfaktor vermischt. Da es sich bei den meisten Blutgerinnungsfaktoren um Serin-Endopeptidasen handelt, also um Hydrolasen, die Peptidbindungen spalten können, werden vorwiegend Peptidsubstrate verwendet, die möglichst spezifisch von dem zu bestimmenden Blutgerinnungsfaktor gespalten werden und die eine nachweisbare Signalgruppe aufweisen. Bevorzugterweise werden abspaltbare chromogene oder fluorogene Signalgruppen verwendet, die photometrisch bestimmt werden. In den Patentdokumenten EP 34122 A1 und US 4,508,644 sind eine Vielzahl von chromogenen Peptidsubstraten und deren Verwendung in gerinnungsdiagnostischen Tests beschrieben, z. B. zur Bestimmung der Gerinnungsfaktoren Faktor IIa (Thrombin) und Xa. In dem Dokument EP 78764 A1 ist ein chromogenes Verfahren zur Bestimmung des Gerinnungsfaktors XIIa beschrieben.

Insbesondere mit Hilfe der chromogenen Teste können auch Antikoagulanzien, die die Aktivität von Blutgerinnungsfaktoren inhibieren, in Patientenproben bestimmt werden. Dazu wird die zu untersuchende Patientenprobe mit einem aktivierten Gerinnungsfaktor und mit einem Substrat für diesen Gerinnungsfaktor vermischt. Je mehr Antikoagulanz in der Probe enthalten ist, desto stärker wird der aktivierte Gerinnungsfaktor inhibiert und desto weniger Substrat wird gespalten.

Bei diesen bekannten Verfahren handelt es sich um homogene Verfahren. Homogene Testverfahren haben den Vorteil, dass die Probe mit den Nachweisreagenzien zu einem Testansatz vermischt wird und die Nachweisreaktion in diesem Testansatz gemessen wird, ohne dass zusätzliche Separationsschritte, zum Beispiel zum Abtrennen des Analyten von übrigen Probenbestandteilen, notwendig sind. Homogene Testverfahren haben jedoch auch den Nachteil, dass probenintrinsische Substanzen während des gesamten Testverfahrens anwesend sind und dadurch die Nachweisreaktion beeinflussen oder die Messung der Nachweisreaktion stören können. Patientenproben können zum Beispiel in Einzelfällen abnormal hohe Konzentrationen einer oder mehrerer intrinsischer, also körpereigener Substanzen enthalten, die sich bei Überschreitung einer tolerablen Konzentration in photometrischen Detektionsverfahren als störend erweisen und sich zu einem systematischen Fehler auswirken können. Probleme bereiten bekanntermaßen hämolytische, ikterische und/oder lipämische Serum- oder Plasmaproben, sogenannte HIL-Proben, die über abnormal hohe Hämoglobin-, Bilirubin- und/oder Triglyzerid-Konzentrationen verfügen. Abnormal hohe Konzentrationen dieser interferierenden Substanzen können durch einen pathologischen Zustand des Patienten oder aber durch eine unsachgemäße Probengewinnung oder -lagerung verursacht werden.

Die vorliegende Anmeldung beschreibt ein Verfahren zur Bestimmung der Aktivität von proteolytischen Gerinnungsfaktoren in einer Probe, das weniger störanfällig gegenüber probenintrinsischen Störsubstanzen ist, und das folgende Schritte umfasst:
a. Bereitstellen und Inkubation eines Reaktionsgemisches enthaltend
   i. die Probe,
   ii. ein Agens zur direkten oder indirekten Aktivierung des proteolytischen Gerinnungsfaktors in der Probe,
   iii. ein spaltbares Substrat, welches mindestens eine Spaltstelle für den aktivierten Gerinnungsfaktor aufweist,
   iv. eine Festphase, an die das spaltbare Substrat gebunden ist oder während der Inkubation gebunden wird;
b. Abtrennung der Festphase; und
c. Bestimmung der Menge von Festphasen-gebundenem, ungespaltenem Substrat.

Die Menge von Festphasen-gebundenem, ungespaltenem Substrat verhält sich umgekehrt proportional zur Aktivität des zu bestimmenden proteolytischen Gerinnungsfaktors.

Das Bereitstellen des Reaktionsgemisches umfasst immer das In-Kontakt-Bringen der Probe, bevorzugterweise einer Blut- oder Plasmaprobe, mit einem Agens zur direkten oder indirekten Aktivierung des zu bestimmenden proteolytischen Gerinnungsfaktors in der Probe und mit einer Festphase. Das spaltbare Substrat, welches mindestens eine Spaltstelle für den aktivierten Gerinnungsfaktor aufweist, kann dem Reaktionsgemisch auf unterschiedliche Art und Weise zugeführt werden:
- Das spaltbare Substrat, beispielsweise ein synthetisches Peptid oder ein gereinigtes Protein, welches nicht an eine Festphase gebunden ist, kann in einem separaten Reagenz enthalten sein, welches dem Reaktionsgemisch zugegeben wird. Das Reaktionsgemisch enthält dann eine Festphase, an die das spaltbare Substrat während der Inkubation gebunden wird.
- Das spaltbare Substrat, beispielsweise ein synthetisches Peptid oder ein gereinigtes Protein, ist bereits an die Festphase gebunden und wird zusammen mit der Festphase als Bestandteil derselben mit der Probe und dem Agens zur Aktivierung des proteolytischen Gerinnungsfaktors in Kontakt gebracht.
- Das spaltbare Substrat ist ein natürlicherweise in der Probe enthaltenes, natürliches Substrat für den aktivierten Gerinnungsfaktor und wird dadurch als Bestandteil des Probenmaterials mit der Probe dem Reaktionsgemisch zugeführt. Das Reaktionsgemisch enthält dann eine Festphase, an die das spaltbare natürliche Substrat während der Inkubation gebunden wird.

Spaltbare Substrate, welche mindestens eine Spaltstelle für einen aktivierten Gerinnungsfaktor aufweisen, sind dem Fachmann hinlänglich bekannt. Ein spaltbares Substrat kann ein synthetisch, rekombinant oder biotechnologisch hergestelltes Molekül oder ein natürliches Molekül sein, das durch Einwirken des aktivierten Gerinnungsfaktors in zwei Spaltprodukte zerlegt wird. Ein spaltbares Substrat kann ganz oder teilweise aus einem Peptid bestehen. Bevorzugterweise umfasst es zumindest im Bereich der Spaltstelle einen Peptidanteil. Bevorzugterweise besteht der Peptidanteil eines spaltbaren Substrats aus 3 bis etwa 150 Aminosäureresten.

In einer anderen Ausführungsform kann das spaltbare Substrat aus einem vollständigen Protein bestehen oder aus einem Proteinfragment. Ein spaltbares Substrat kann auch ein natürliches Substrat eines aktivierten Gerinnungsfaktors sein. Ein Beispiel für ein natürliches spaltbares Substrat ist Faktor V, der Spaltstellen für aktiviertes Protein C, Faktor Xa, Faktor IIa (Thrombin) und Plasmin aufweist. Ein weiteres Beispiel ist Fibrinogen, welches Spaltstellen für Faktor IIa (Thrombin) aufweist. Noch ein weiteres Beispiel ist Faktor II (Prothrombin), welches Spaltstellen für Faktor IIa (Thrombin), Faktor Xa und diverse Schlangengifte, wie z. B. Ecarin oder Textarin aufweist.

In einer Ausführungsform des Verfahrens ist das spaltbare Substrat, welches mindestens eine Spaltstelle für den aktivierten Gerinnungsfaktor aufweist, an eine Festphase gebunden.

Der Begriff "gebunden" ist breit zu verstehen und umfasst beispielsweise eine kovalente und eine nicht-kovalente Bindung, eine direkte und eine indirekte Bindung, die Adsorption an eine Oberfläche und den Einschluß in eine Vertiefung oder einen Hohlraum, etc. Bei einer kovalenten Bindung ist das spaltbare Substrat über eine chemische Bindung an die Festphase gebunden. Beispiele für eine nicht-kovalente Bindung sind die Oberflächenadsorption, der Einschluss in Hohlräume oder die Bindung von zwei spezifischen Bindungspartnern. Neben einer direkten Bindung an die Festphase kann das spaltbare Substrat auch indirekt über spezifische Wechselwirkung mit anderen spezifischen Bindungspartnern an die Festphase gebunden sein.

In einer bevorzugten Ausführungsform weist das spaltbare Substrat einen ersten Bindungspartner A eines ersten Bindungspaares A/B auf, und die Festphase weist den Bindungspartner B auf, und das Substrat ist durch Bindung der Bindungspartner A und B an die Festphase gebunden.

In einer anderen Ausführungsform des Verfahrens wird das spaltbare Substrat, welches mindestens eine Spaltstelle für den aktivierten Gerinnungsfaktor aufweist, während der Inkubation des Reaktionsgemisches an die Festphase gebunden. Dazu weist das spaltbare Substrat einen ersten Bindungspartner A eines ersten Bindungspaares A/B auf, und die Festphase weist den Bindungspartner B auf, und das Substrat wird während der Inkubation des Reaktionsgemisches durch Bindung der Bindungspartner A und B an die Festphase gebunden.

Geeignete Bindungspaare A/B sind vor allem Antigen/Antikörper-Kombinationen, wobei der Bindungspartner A ein antigenes Epitop des spaltbaren Substrats ist. Das antigene Epitop kann ein natürliches Sequenz- oder Strukturepitop eines natürlichen Proteins oder Proteinfragmentes sein, insbesondere dann, wenn als spaltbares Substrat ein natürliches, in der Probe enthaltenes Substrat verwendet wird. Das antigene Epitop kann auch ein heterologes Sequenz- oder Strukturepitop eines modifizierten spaltbaren Substrates sein. Beispiele für heterologe Sequenz- oder Strukturepitope sind FLAG-, oder HIS- oder Fluorescein-Tags, die inbesondere zur Markierung von Peptiden oder Proteinen verwendet werden. Der Festphasen-gebundene Bindungspartner B ist so zu wählen, dass das spaltbare Substrat spezifisch gebunden werden kann. Bevorzugterweise besteht der Bindungspartner B aus einem Antikörper oder einem Antigen-bindenden Fragment desselben. Besonders bevorzugte Bindungspaare A/B sind FLAG-Tag/anti-FLAG-Tag-Antikörper, HIS-Tag/anti-HIS-Tag-Antikörper und Fluorescein/anti-Fluorescein-Antikörper.

In einer weiteren Ausführungsform des Verfahrens weist das spaltbare Substrat einen ersten Bindungspartner X eines zweiten Bindungspaares X/Y auf, der mit dem zweiten Bindungspartner Y des zweiten Bindungspaares X/Y wechselwirkt und wobei der zweite Bindungspartner Y mit einer Komponente eines signalbildenden Systems assoziiert ist. Auf diese Weise kann ungespaltenes Substrat nachgewiesen werden. Der Bindungspartner X ist in einem Bereich des Substrats angeordnet, der, sofern das Substrat von dem aktivierten proteolytischen Gerinnungsfaktor gespalten wird, von dem Festphasen-gebundenen Bereich des Substrats abgetrennt wird.

Geeignete Bindungspaare X/Y sind zum Beispiel Antigen/Antikörper-Kombinationen, wobei der Bindungspartner X ein antigenes Epitop des spaltbaren Substrats ist. Das antigene Epitop kann ein natürliches Sequenz- oder Strukturepitop eines natürlichen Proteins oder Proteinfragmentes sein, insbesondere dann, wenn als spaltbares Substrat ein natürliches, in der Probe enthaltenes Substrat verwendet wird. Das antigene Epitop kann auch ein heterologes Sequenz- oder Strukturepitop eines modifizierten spaltbaren Substrates sein. Beispiele für heterologe Sequenz- oder Strukturepitope sind FLAG-, oder HIS- oder Fluorescein-Tags, die inbesondere zur Markierung von Peptiden oder Proteinen verwendet werden. Weitere geeignete Bindungspaare X/Y sind zum Beispiel Biotin/Avidin und Biotin/Streptavidin.

Der zweite Bindungspartner Y ist mit einer Komponente eines signalbildenden Systems assoziiert.

Bei einem "signalbildenden System" kann es sich um eine oder mehrere Komponenten handeln, wobei es sich wenigstens bei einer Komponente um ein nachweisbares Label handelt. Als Label ist jedes Molekül zu verstehen, das selbst ein Signal - produziert oder die Produktion eines Signals induzieren kann, wie z.B, eine fluoreszierende Substanz, eine radioaktive Substanz, ein Enzym oder eine chemilumineszierende Substanz. Das Signal kann beispielsweise anhand der Enzymaktivität, der Lumineszenz, der Lichtabsorption, der Lichtstreuung, der ausgestrahlten elektromagnetischen oder radioaktiven Strahlung oder einer chemischen Reaktion nachgewiesen oder gemessen werden.

Ein Label vermag selbst ein nachweisbares Signal zu erzeugen, so dass keine weiteren Komponenten notwendig sind. Viele organische Moleküle absorbieren ultraviolettes und sichtbares Licht, wodurch diese Moleküle in einen angeregten Energiezustand kommen können und die absorbierte Energie in Form von Licht einer anderen Wellenlänge als der des eingestrahlten Lichts abgeben. Wieder andere Label können direkt ein nachweisbares Signal erzeugen wie z.B. radioaktive Isotope oder Farbstoffe.

Wieder andere Label benötigen zur Signalerzeugung weitere Komponenten, d. h. das signalproduzierende System schließt in einem solchen Fall all die für die Signalbildung benötigten Komponenten mit ein wie z. B. Substrate, Coenzyme, Quencher, Beschleuniger, zusätzliche Enzyme, Substanzen, die mit Enzymprodukten reagieren, Katalysatoren, Aktivatoren, Cofaktoren, Inhibitoren, Ionen etc.

Geeignete Label sind beispielsweise Enzyme einschließlich Meerrettichperoxidase, alkalische Phosphatase, Glukose-6-Phosphatdehydrogenase, Alkoholdehydrogenase, Glucoseoxidase, β -Galactosidase, Luciferase, Urease und Acetylcholinesterase; Farbstoffe; fluoreszierende Substanzen einschließlich Fluoresceinisothiocyanat, Rhodamin, Phycoerythrin, Phycocyanin, Ethidiumbromid, 5-Dimethylaminonapthalen-1-sulfonylchlorid und fluoreszierende Chelate von seltenen Erden; chemilumineszierende Substanzen einschließlich Luminol, Isoluminol, Acridiniumverbindungen, Olefin, Enolether, Enamin, Arylvinylether, Dioxen, Arylimidazol, Lucigenin, Luciferin und Aequorin; Sensitizer einschließlich Eosin, 9,10-Dibromoanthracen, Methylen Blau, Porphyrin, Phthalocyanin, Chlorophyll, Rose Bengal; Coenzyme; Enzymsubstrate; radioaktive Isotope einschließlich ¹²⁵I, ¹³¹I, ¹⁴C, 3H, ³²P, ³³P, ³⁵S, ⁵¹Cr, ⁵⁹ Fe, ⁵⁷Co und ⁷⁵Se.

Die Festphase, an die das spaltbare Substrat gebunden ist oder während der Inkubation gebunden wird, beinhaltet einen Gegenstand, der aus porösem und/oder nicht porösem, in der Regel wasserunlöslichem Material besteht und die unterschiedlichsten Formen aufweisen kann, wie z. B. die von Gefäßen, Röhrchen, Mikrotitrationsplatten, Kugeln, Mikropartikeln, Stäbchen, Streifen, Filter- oder Chromatographiepapier, etc. In der Regel ist die Oberfläche der Festphase hydrophil oder kann hydrophil gemacht werden. Die Festphase kann aus den unterschiedlichsten Materialien bestehen, wie z. B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere, wie z.B. Zellulose, Nitrozellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Keramik, Glas, Metalle, insbesondere Edelmetalle wie Gold und Silber; Magnetit; Mischungen oder Kombinationen derselben; etc.

Die Festphase kann einen Überzug aus einer oder mehreren Schichten aufweisen z. B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkende Agenzien.

Zur direkten oder indirekten Aktivierung des proteolytischen Gerinnungsfaktors in der Probe wird die Probe üblicherweise mit einem Agens vermischt, das eine direkte oder indirekte Aktivierung des proteolytischen Gerinnungsfaktors bewirkt. Unter direkter Aktivierung ist zu verstehen, dass ein Agens verwendet wird, das den zu bestimmenden proteolytischen Gerinnungsfaktor direkt aktiviert, unabhängig von der Anwesenheit anderer Gerinnungsfaktoren. Unter indirekter Aktivierung ist zu verstehen, dass ein Agens verwendet wird, das einen oder mehrere Blutgerinnungsfaktoren der Blutgerinnungskaskade aktiviert, welche wiederum den zu untersuchenden proteolytischen Gerinnungsfaktor aktivieren. Die Art des Agens hängt davon ab, welcher Gerinnungsfaktor bestimmt werden soll, ob die Aktivität des Gerinnungsfaktors allein bestimmt werden soll oder ob die Funktionalität der Blutgerinnungskaskade oder eines Teilbereichs der Blutgerinnungskaskade (extrinsischer oder intrinsischer Weg) anhand eines Gerinnungsfaktors bestimmt werden soll. Substanzen und spezifische Mischungen verschiedener Substanzen, die eine direkte oder indirekte Aktivierung von proteolytischen Gerinnungsfaktoren ermöglichen, sind dem Fachmann hinlänglich bekannt und umfassen beispielsweise Phospholipide, wie z. B. negativ geladene Phospholipide; Lipoproteine, wie z. B. Thromboplastin; Proteine, wie z. B. Gewebefaktor, aktivierte Serinproteasen, wie z. B. Faktor IIa (Thrombin), Faktor VIIa, Faktor IXa, Faktor Xa, Faktor XIa, Faktor XIIa oder aktiviertes Protein C; Schlangengifte, wie z. B. PROTAC^{®} Enzym, Ecarin, Textarin, Noscarin, Batroxobin, Thrombocytin oder Russell's Viper Venom (RVV); Kontaktaktivatoren, wie z. B. Silica, Kaolin, Ellagsäure oder Celite. Weitere Substanzen, die ein Agens enthalten kann, sind z. B. Puffersubstanzen, Salze, Detergenzien, Ionen, insbesondere Calciumionen und Chelatbildner.

Unter einer "Probe" ist im Sinne der Erfindung das Material zu verstehen, das den nachzuweisenden proteolytischen Gerinnungsfaktor vermutlich enthält. Der Begriff Probe umfasst insbesondere menschliche oder tiereische Körperflüssigkeiten, vornehmlich Blut und Plasma.

Nachdem das Reaktionsgemisch, das die Probe, ein Agens zur Aktivierung des proteolytischen Gerinnungsfaktors, ein spaltbares Substrat und eine Festphase, an die das spaltbare Substrat gebunden ist oder gebunden wird, enthält, bereit gestellt ist, wird das Reaktionsgemisch für eine begrenzte Zeit inkubiert, um eine ausreichende Aktivierung des Gerinnungsfaktors, eine ausreichende Spaltung des Substrats durch den aktivierten Gerinnungsfaktor und gegebenenfalls eine ausreichende Bindung des spaltbaren Substrats bzw. des Spaltprodukts des Substrats an die Festphase zu gewährleisten. Der Begriff "ausreichend" ist so zu verstehen, dass das Verfahren als Ganzes eine quantitative Bestimmung der Aktivität des Gerinnungsfaktors ermöglicht. Die optimale Inkubationsdauer eines bestimmten Testaufbaus kann experimentell ermittelt werden.

In einer bevorzugten Ausführungsform, kann dem Reaktionsgemisch ferner ein Inhibitor der Fibrinaggregation zugegeben werden. Unter einem Fibrinaggregationsinhibitor ist eine Substanz, insbesondere ein synthetisches Oligopeptid, zu verstehen, das die Aneinanderlagerung (Polymerisation) der Fibrinmonomere, die unter der Einwirkung von Thrombin entstehen, inhibiert und damit eine Gerinnselbildung im Reaktionsgemisch verhindert (siehe z. B. EP 0 456 152 B1).

Nachdem das Reaktionsgemisch inkubiert wurde, wird die Festphase und damit die daran gebundenen Bestandteile von den übrigen Bestandteilen des Reaktionsgemisches abgetrennt. Die Abtrennung kann je nach Art der Festphase auf unterschiedliche Art und Weise erfolgen, z. B. durch Zentrifugation, Filtration, magnetische Abtrennung oder durch Absaugen der flüssigen Phase des Reaktionsgemisches. Nach der Abtrennung der Festphase kann mindestens ein Waschschritt durchgeführt werden, um Rückstände des Reaktionsgemisches möglichst vollständig von der Festphase zu entfernen und/oder um die Festphase für die nachfolgende Nachweisreaktion vorzubereiten. Dazu wird die Festphase mit einer Waschlösung, bevorzugterweise mit einer Pufferlösung, inkubiert und anschließend von der Waschlösung wieder abgetrennt.

Die Bestimmung der Menge von Festphasen-gebundenem ungespaltenem Substrat kann je nach Art des verwendeten Nachweissystems auf unterschiedliche Art und Weise erfolgen, z. B. durch Inkubation der Festphase mit einem Reagenz, das Substanzen enthält, welche spezifisch mit dem ungespaltenen Substrat wechselwirken und ein messbares Signal erzeugen. Bevorzugterweise erfolgt die Bestimmung der Menge von Festphasen-gebundenem ungespaltenem Substrat dadurch, dass die Festphase mit einem Nachweisreagenz inkubiert wird, das den Bindungspartner Y des zweiten Bindungspaares X/Y enthält, der spezifisch an den Bindungspartner X des ungespaltenen Substrats bindet. Der Bindungspartner Y kann entweder direkt mit einer signalgebenden Kompenente assoziiert sein oder mit einer signalgebenden Kompenente assoziiert werden.

Die Menge oder Stärke des Signals verhält sich proportional zu der Menge von Festphasen-gebundenem ungespaltenem Substrat und damit umgekehrt proportional zur Aktivität des proteolytischen Gerinnungsfaktors.

Das Verfahren zur Bestimmung eines proteolytischen Gerinnungsfaktors eignet sich insbesondere zur Bestimmung der proteolytischen Gerinnungsfaktoren Faktor II, Faktor VII, Faktor IX, Faktor X, Faktor XI, Faktor XII oder Protein C.

Der vorliegenden Erfindung lag die Aufgabe zugrunde, ein Verfahren zur quantitativen Bestimmung eines Antikoagulanzes in einer Probe bereit zu stellen, das weniger anfällig gegenüber probenintrinsischen Störsubstanzen ist.

Diese Aufgabe wird erfindungsgemäß dadurch gelöst, dass das Verfahren folgende Schritte umfasst:
a. Bereitstellen und Inkubation eines Reaktionsgemisches enthaltend
   i. die Probe,
   ii. eine definierte Menge eines aktivierten Gerinnungsfaktors, dessen Aktivität direkt oder indirekt von dem zu bestimmenden Antikoagulanz beeinflussbar ist, und wobei der aktivierte Gerinnungsfaktor in einem separaten Reagenz enthalten ist, welches dem Reaktionsgemisch zugegeben wird,
   iii. ein spaltbares Substrat, welches mindestens eine Spaltstelle für den aktivierten Gerinnungsfaktor aufweist,
   iv. eine Festphase, an die das spaltbare Substrat gebunden ist oder während der Inkubation gebunden wird;
b. Abtrennung der Festphase; und
c. Bestimmung der Menge von Festphasen-gebundenem, ungespaltenem Substrat.

Bei diesem Verfahren wird eine bekannte, definierte Menge eines aktivierten Gerinnungsfaktors zu dem Reaktionsgemisch gegeben. Welcher aktivierte Gerinnungsfaktor zugegeben wird, hängt davon ab, welches Antikoagulanz bestimmt werden soll.

Für die Bestimmung eines Heparins, d. h. eines hochmolekularen, unfraktionierten Heparins (HMW Heparin) oder eines niedermolekularen Heparins (LMW Heparin) oder eines Heparinoids, eignet sich insbesondere die Zugabe von Faktor IIa (Thrombin) oder von Faktor Xa. Für die Bestimmung eines direkten Thrombininhibitors, z. B. von Argatroban, Melagatran, Ximelagatran, Bivalirudin, Dabigatran oder Hirudin, eignet sich insbesondere die Zugabe von Faktor IIa (Thrombin). Für die Bestimmung eines direkten Faktor Xa-Inhibitors, z. B. von Rivaroxaban, eignet sich insbesondere die Zugabe von Faktor Xa.

Je mehr Antikoagulanz in der Probe enthalten ist, desto stärker wird der zugegebene aktivierte Gerinnungsfaktor inhibiert und desto weniger des spaltbaren Substrats wird gespalten. Die Menge von Festphasen-gebundenem, ungespaltenem Substrat verhält sich daher proportional zu der in der Probe enthaltenen Menge oder Aktivität des zu bestimmenden Antikoagulanzes.

Abgesehen davon, dass bei dem erfindungsgemäßen Verfahren zur quantitativen Bestimmung eines Antikoagulanzes
- eine definierte Menge eines aktivierten Gerinnungsfaktors, dessen Aktivität direkt oder indirekt von dem zu bestimmenden Antikoagulanz beeinflussbar ist, und wobei der aktivierte Gerinnungsfaktor in einem separaten Reagenz enthalten ist, welches dem Reaktionsgemisch zugegeben wird, und dass
- daher das Reaktionsgemisch kein Agens zur Aktivierung eines proteolytischen Gerinnungsfaktors enthalten muss, treffen die obigen Erläuterungen zur Durchführung des Verfahrens zur Bestimmung eines proteolytischen Gerinnungsfaktors auch auf das erfindungsgemäße Verfahren zur quantitativen Bestimmung eines Antikoagulanzes zu.

Die folgenden Beispiele dienen der Veranschaulichung der vorliegenden Erfindung und sind nicht als Einschränkung zu verstehen.

### Figurenbeschreibung

**Figur 1**

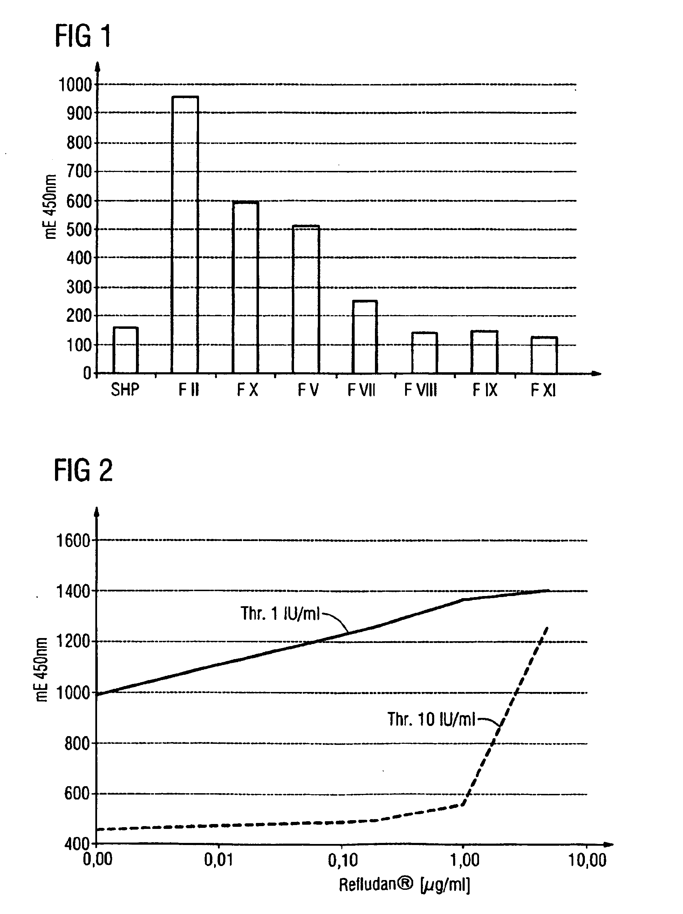

   Figur 1 zeigt die Extinktionswerte bei 450 nm von Proben, bei denen der extrinsische Weg der Gerinnung durch Innovin^{®} (ein Thromboplastinreagenz) aktiviert wurde und bei denen die Thrombinaktivität bestimmt wurde (siehe Beispiel 1). Bei den Proben handelt es sich um Standard Human Plasma (SHP), welches in vollem Umfang alle Gerinnungsfaktoren enthält, sowie Plasmen mit Mangel an Faktoren des extrinsischen (F II, F V, F VII, F X) bzw. des intrinsischen Gerinnungswegs (F VIII, F IX, F XI). In Abhängigkeit von der Menge an Thrombin in der aktivierten Probe, die wiederum vom Vorhandensein der Faktoren des extrinsischen Gerinnungswegs abhängig ist, wird das Thrombin-sensitive Peptidsubstrat gespalten. Je mehr Thrombin in der Probe enthalten ist, desto mehr Thrombinsubstrat wird gespalten und desto weniger ungespaltenes Thrombinsubstrat kann nachgewiesen werden. Die gemessenen Extinktionswerte sind somit umgekehrt proportional zur Thrombinaktivität in der Probe bzw. zur Aktivität der extrinsischen Gerinnungskaskade. In dem gezeigten Experiment weisen daher alle Plasmen mit Mangel an Faktoren des extrinsischen Gerinnungsweges (insbesondere das F II-Mangelplasma) höhere Extinktionswerte als Normalplasma (SHP) auf, wohingegen Plasmen mit am extrinsischen Gerinnungsweg unbeteiligten Faktoren (Faktor VIII, IX, XI) keine Signalunterschiede zu SHP aufweisen. Daher konnte mit dem gezeigten Test ein Mangel an Faktoren des extrinsischen Gerinnungsweges in Plasmen eindeutig nachgewiesen werden.
**Figur 2**
   Figur 2 zeigt die Extinktionswerte bei 450 nm von Proben mit unterschiedlicher Refludan^{®}-Konzentration, denen eine definierte Mengen Thrombin zugesetzt wurden (Thr. 1 IU/ml, Thr. 10 IU/ml) und bei denen die Thrombinaktivität bestimmt wurde (siehe Beispiel 2). Je mehr von dem direkten Thrombininhibitor Refludan^{®} in einer Probe enthalten ist, desto mehr von dem zugesetzten Thrombin wird inhibiert, desto weniger Thrombinsubstrat wird gespalten und desto mehr ungespaltenes Thrombinsubstrat kann nachgewiesen werden. Die gemessenen Extinktionswerte sind somit proportional zur Thrombin-inhibierenden Aktivität des Refludan^{®} in der Probe. Dabei wurde deutlich, dass die Sensitivität des Verfahrens für Proben mit therapeutisch niedrigen Refludan^{®}-Konzentrationen (bis 1 µg/ml) bei Zusatz von einer vergleichsweise niedrigen Thrombinmenge (1 IU/ml) besonders gut ist, während die Sensitivität des Verfahrens für Proben mit therapeutisch hohen Refludan^{®}-Konzentrationen (1-5 µg/ml) bei Zusatz von einer vergleichsweise hohen Thrombinmenge (10 IU/ml) besonders gut ist.

### Beispiele

### Beispiel 1: Verfahren zur Bestimmung der Thrombinaktivität

Zunächst wurden Mikrotiterplatten (Nunc-Thermofisher, Roskilde, Dänemark) mit einem monoklonalen FLAG-Epitopspezifischen Antikörper (MAK M2, Sigma Aldrich, München, Deutschland) beschichtet. Anschließend wurde ein sieben Aminosäurereste umfassendes Thrombin-spezifisches Peptidsubstrat mit den Aminosäureresten, Leucin, Valin, Prolin, Arginin, Glycin, Phenylalanin, Glycin in der genannten Sequenz, welches am aminoterminalen Ende ein FLAG-Tag-Epitop aufweist und am carboxyterminalen Ende biotinyliert ist, mit dem Festphasen-gebundenen anti-FLAG-Antikörper inkubiert und so an die Festphase gebunden. Pro Kavität wurden anschließend in folgender Reihenfolge folgende Reagenzien zugegeben:

25 µl einer Fibrinaggregationsinhibitor-Lösung (6 mg/ml eines synthetischen Oligopeptids mit den Aminosäureresten Glycin, Prolin, Arginin, Prolin, Alanin in der genannten Sequenz) ; 25 µl Probe; und 25 µl Innovin^{®} (rekombinanter humaner Tissue Factor mit synthetischen Phospholipiden, Siemens Healthcare Diagnostics, Marburg, Deutschland) als Gerinnungsaktivator.

Als Proben wurden Standard Human Plasma(SHP) beziehungsweise Gerinnungsfaktor-Mangelplasmen (Faktor II, Faktor X, Faktor V, Faktor VII, Faktor VIII, Faktor IX, Faktor XI) eingesetzt.

Das Reaktionsgemisch wurde durchmischt und für 15 Minuten bei 37 °C inkubiert. Dann wurde das Reaktionsgemisch abgesaugt, und jede Kavität wurde dreimal mit jeweils 250 µl Waschpuffer gewaschen. Zum Nachweis von ungespaltenem, Festphasen-gebundenem Thrombin-Substrat wurden in jede Kavität 100 µl einer Streptavidin/Peroxidase (POD)-Konjugatlösung (0,33 µg/ml, Sigma Aldrich) zugegeben und wiederum für 30 Minuten bei 20-25 °C inkubiert. Dann wurde die Konjugatlösung abgesaugt, und jede Kavität wurde dreimal mit jeweils 250 µl

Waschpuffer gewaschen. Anschließend wurden in jede Kavität 100 µl einer Pufferlösung mit 0,5 g/L TMB (3,3'5,5'-Tetramethylbenzidin-dihydrochlorid) und 0,1g/L Wasserstoffperoxid zugegeben und wiederum für 30 Minuten bei 20-25 °C inkubiert. Zum Abstoppen der Peroxidase-Reaktion wurden in jede Kavität 100 µl 0,5 N Schwefelsäure gegeben, und es wurde die Extinktion bei 450 nm abzüglich der Referenzwellenlänge 650 nm mittels eines MTP-Photometers Sunrise^{®} (Tecan Trading AG, Schweiz) bestimmt.

Die Ergebnisse sind in Figur 1 dargestellt.

### Beispiel 2: Verfahren zur quantitativen Bestimmung des direkten Thrombininhibitors Refludan^{®}

Wie in Beispiel 1 beschrieben, wurde ein sieben Aminosäurereste umfassendes Thrombin-spezifisches Peptidsubstrat, welches am aminoterminalen Ende ein FLAG-Tag-Epitop aufweist und am carboxyterminalen Ende biotinyliert ist, über einen anti-FLAG-Antikörper an die Festphase (Mikrotiterplatte) gebunden.

Pro Kavität wurden in folgender Reihenfolge folgende Reagenzien zugegeben:
50 µl einer Fibrinaggregationsinhibitor-Lösung (3 mg/ml eines synthetischen Oligopeptids mit den Aminosäureresten Glycin, Prolin, Arginin, Prolin, Alanin in der genannten Sequenz);
25 µl Probe; und
25 µl Rinder-α-Thrombinlösung (4 oder 40 IU Rinder-α-Thrombin/ml, 10 KIE/ml Aprotinin, 150 mM/L NaCl, 5 mg/ml Bovines Serumalbumin, 10 mg/ml Mannit, 5 µg/ml Hexadimethrinbromid).

Durch die unterschiedlichen Thrombinkonzentrationen kann die Sensitivität in den verschiedenen therapeutischen Bereichen gewährleistet werden.

Als Proben wurden Standard Human Plasma(SHP) beziehungsweise normale humane Citratplasmaproben verwendet. Diese wurden aliquotiert, und zu den Aliquots wurden jeweils 0,0 µg/ml, 0,2 µg/ml, 1,0 µg/ml und 5 µg/ml Refludan^{®}(Lepirudin, rekombinantes Hirudin,CSL Behring GmbH, Marburg, Deutschland), zugegeben.

Der Nachweis von ungespaltenem, Festphasen-gebundenem Thrombin-Substrat wurde, wie in Beispiel 1 beschrieben, durchgeführt, und es wurde die Extinktion bei 450 nm abzüglich der Referenzwellenlänge 650 nm mittels einem MTP-Photometer Sunrise^{®} (Tecan Trading AG, Schweiz) bestimmt.

Die Ergebnisse sind in Figur 2 dargestellt.

## Patentansprüche

1. Verfahren zur quantitativen Bestimmung eines Antikoagulanzes in einer Probe, wobei das Verfahren folgende Schritte umfasst:
a. Bereitstellen und Inkubation eines Reaktionsgemisches enthaltend
i. die Probe,
ii. eine definierte Menge eines aktivierten Gerinnungsfaktors, dessen Aktivität direkt oder indirekt von dem zu bestimmenden Antikoagulanz beeinflussbar ist, und wobei der aktivierte Gerinnungsfaktor in einem separaten Reagenz enthalten ist, welches dem Reaktionsgemisch zugegeben wird,
iii. ein spaltbares Substrat, welches mindestens eine Spaltstelle für den aktivierten Gerinnungsfaktor aufweist,
iv. eine Festphase, an die das spaltbare Substrat gebunden ist oder während der Inkubation gebunden wird;
b. Abtrennung der Festphase; und
c. Bestimmung der Menge von Festphasen-gebundenem, ungespaltenem Substrat.

2. Verfahren nach Anspruch 1 zur quantitativen Bestimmung eines Antikoagulanzes aus der Gruppe LMW Heparin, HMW Heparin, Heparinoide, direkte Thrombininhibitoren und direkte Faktor Xa-Inhibitoren.

3. verfahren nach Anspruch 1, wobei das Reaktionsgemisch eine definierte Menge eines aktivierten Gerinnungsfaktors aus der Gruppe Faktor IIa und Faktor Xa enthält.

4. Verfahren nach Anspruch 1, wobei das spaltbare Substrat in einem separaten Reagenz enthalten ist, welches dem Reaktionsgemisch zugegeben wird und wobei das Reaktionsgemisch eine Festphase enthält, an die das spaltbare Substrat während der Inkubation gebunden wird.

5. Verfahren nach Anspruch 4, wobei das spaltbare Substrat ein Peptid umfasst, welches aus 3 bis 150 Aminosäureresten besteht.

6. Verfahren nach Anspruch 1, wobei das spaltbare Substrat ein natürliches Substrat für den aktivierten Gerinnungsfaktor und ein Bestandteil der Probe ist und wobei das Reaktionsgemisch eine Festphase enthält, an die das spaltbare Substrat während der Inkubation gebunden wird.

7. Verfahren nach Anspruch 1, wobei das Reaktionsgemisch eine Festphase enthält, an die das spaltbare Substrat kovalent gebunden ist.

8. Verfahren nach Anspruch 1, wobei das spaltbare Substrat einen ersten Bindungspartner A eines ersten Bindungspaares A/B aufweist und wobei die Festphase den zweiten Bindungspartner B des ersten Bindungspaares A/B aufweist und wobei das spaltbare Substrat durch Bindung der Bindungspartner A und B an die Festphase gebunden ist oder während der Inkubation gebunden wird.

9. Verfahren nach Anspruch 8, wobei die Bindungspartner A und B so gewählt sind, dass sie ein Bindungspaar A/B aus der Gruppe FLAG-Tag/anti-FLAG-Tag-Antikörper, HIS-Tag/anti-HIS-Tag-Antikörper, Fluorescein/anti-Fluorescein-Antikörper bilden.

10. Verfahren nach einem der vorhergehenden Ansprüche, wobei das spaltbare Substrat einen ersten Bindungspartner X eines zweiten Bindungspaares X/Y aufweist und wobei der zweite Bindungspartner Y des zweiten Bindungspaares X/Y mit einer Komponente eines signalbildenden Systems assoziiert ist.

11. Verfahren nach Anspruch 10 wobei die Bindungspartner X und Y so gewählt sind, dass sie ein Bindungspaar X/Y aus der Gruppe Biotin/Avidin, Biotin/Streptavidin bilden.

12. Verfahren nach einem der vorhergehenden Ansprüche, wobei dem Reaktionsgemisch ferner ein Inhibitor der Fibrinaggregation zugegeben wird.

## Claims

1. A method for quantitatively determining an anticoagulant in a sample, wherein the method comprises the following steps:
a. providing and incubating a reaction mixture comprising
i. the sample,
ii. a defined amount of an activated coagulation factor whose activity is directly or indirectly influenceable by the anticoagulant to be determined, wherein the activated coagulation factor is present in a separate reagent which is added to the reaction mixture,
iii. a cleavable substrate which has at least one cleavage site for the activated coagulation factor,
iv. a solid phase to which the cleavable substrate is bound or becomes bound during the incubation;
b. separating the solid phase; and
c. determining the amount of solid-phase-bound, uncleaved substrate.

2. The method as claimed in claim 1 for quantitatively determining an anticoagulant selected from the group consisting of LMW heparin, HMW heparin, heparinoids, direct thrombin inhibitors, and direct factor Xa inhibitors.

3. The method as claimed in claim 1, wherein the reaction mixture comprises a defined amount of an activated coagulation factor selected from the group consisting of factor IIa and factor Xa.

4. The method as claimed in claim 1, wherein the cleavable substrate is present in a separate reagent which is added to the reaction mixture, and wherein the reaction mixture comprises a solid phase to which the cleavable substrate becomes bound during the incubation.

5. The method as claimed in claim 4, wherein the cleavable substrate comprises a peptide which consists of 3 to 150 amino acid residues.

6. The method as claimed in claim 1, wherein the cleavable substrate is a natural substrate for the activated coagulation factor and a constituent of the sample, and wherein the reaction mixture comprises a solid phase to which the cleavable substrate becomes bound during the incubation.

7. The method as claimed in claim 1, wherein the reaction mixture comprises a solid phase to which the cleavable substrate is covalently bonded.

8. The method as claimed in claim 1, wherein the cleavable substrate has a first binding partner A of a first binding pair A/B, and wherein the solid phase has the second binding partner B of the first binding pair A/B, and wherein the cleavable substrate is bound to the solid phase by the binding of binding partners A and B or becomes bound during the incubation.

9. The method as claimed in claim 8, wherein the binding partners A and B are chosen such that they form a binding pair A/B selected from the group consisting of FLAG-tag/anti-FLAG-tag antibody, His-tag/anti-His-tag antibody, fluorescein/anti- fluorescein antibody.

10. The method as claimed in any of the preceding claims, wherein the cleavable substrate has a first binding partner X of a second binding pair X/Y, and wherein the second binding partner Y of the second binding pair X/Y is associated with a component of a signal-producing system.

11. The method as claimed in claim 10, wherein the binding partners X and Y are chosen such that they form a binding pair X/Y selected from the group consisting of biotin/avidin, biotin/streptavidin.

12. The method as claimed in any of the preceding claims, wherein an inhibitor of fibrin aggregation is further added to the reaction mixture.

## Revendications

1. Procédé de détermination quantitative d'un anticoagulant dans un échantillon, le procédé comprenant les stades suivantes :
a. préparation et incubation d'un mélange de réaction contenant
i. l'échantillon
ii. une quantité définie d'un facteur de coagulation activé, dont l'activité peut être influencée directement ou indirectement par l'anticoagulant à déterminer, le facteur de coagulation activé étant contenu dans un réactif distinct, qui est ajouté au mélange de réaction,
iii. un substrat clivable, qui a au moins un point de clivage pour le facteur de coagulation activé,
iv. une phase solide, à laquelle le substrat clivable est fixé ou se fixe pendant l'incubation ;
b. séparation de la phase solide ; et
c. détermination de la quantité de substrat fixé à la phase solide et non clivé.

2. Procédé suivant la revendication 1 de détermination quantitative d'un anticoagulant choisi dans le groupe de l'héparine LMW, de l'héparine HMW, des héparinoïdes, des inhibiteurs directs de la trombine et des inhibiteurs directs du facteur Xa.

3. Procédé suivant la revendication 1, dans lequel le mélange de réaction contient une quantité définie d'un facteur de coagulation activé choisi dans le groupe du facteur IIa et du facteur Xa.

4. Procédé suivant la revendication 1, dans lequel le substrat clivable est contenu dans un réactif distinct, qui est ajouté au mélange de réaction, et le mélange de réaction contient une phase solide, à laquelle le substrat clivable se fixe pendant l'incubation.

5. Procédé suivant la revendication 4, dans lequel le substrat clivable contient un peptide, qui est constitué de 3 à 150 restes d'acide aminé.

6. Procédé suivant la revendication 1, dans lequel le substrat clivable est un substrat naturel pour le facteur de coagulation activé et un constituant de l'échantillon, et le mélange de réaction contient une phase solide, à laquelle le substrat clivable se fixe pendant l'incubation.

7. Procédé suivant la revendication 1, dans lequel le mélange de réaction contient une phase solide, à laquelle le substrat clivable est fixé par covalence.

8. Procédé suivant la revendication 1, dans lequel le substrat clivable a un premier partenaire A de liaison d'une première paire A/B de liaison et la phase solide a le deuxième partenaire B de liaison de la première paire A/B de liaison et le substrat clivable est fixé ou se fixe pendant l'incubation à la phase solide par liaison des partenaires A et B de liaison.

9. Procédé suivant la revendication 8, dans lequel les partenaires A et B de liaison sont choisis, de manière à former une paire A/B de liaison composée du groupe FLAG-Tag/anticorps anti-FLAG-Tag, HIS-Tag/anticorps anti-HIS-Tag, fluorescéine/anticorps anti-fluorescéine.

10. Procédé suivant l'une des revendications précédentes, dans lequel le substrat clivable a un premier partenaire X de liaison d'une deuxième paire X/Y de liaison et le deuxième partenaire Y de liaison de la deuxième X/Y de liaison est associé à un composant d'un système de formation d'un signal.

11. Procédé suivant la revendication 10, dans lequel les partenaires X et Y sont choisis de manière à former une paire X/Y de liaison composée des groupes biotine/avidine, biotine/streptavidine.

12. Procédé suivant l'une des revendications précédentes, dans lequel un inhibiteur de l'agrégation de la fibrine est ajouté, en outre, au mélange de réaction.
